# EUROPEAN PATENT SPECIFICATION

(11) **EP 2 327 384 B1**
(45) Date of publication and mention of the grant of the patent: **21.10.2015**
(21) Application number: 09814426.4
(22) Date of filing: 19.08.2009
(51) Int. Cl.: A61F 13/15, A61F 13/49, A61F 13/496

(54) **DISPOSABLE DIAPER**
WEGWERFWINDEL
COUCHE JETABLE

(30) Priority: 18.09.2008 JP 2008239988
(43) Date of publication of application: 01.06.2011
(73) Proprietor: Unicharm Corporation, Ehime 799-0111 (JP)
(72) Inventor: BABA, Toshimitsu, Kanonji-shi Kagawa 769-1602 (JP); MITSUNO, Satoshi, Kanonji-shi Kagawa 769-1602 (JP); SAITO, Kyota, Kanonji-shi Kagawa 769-1602 (JP); HASHIMOTO, Tatsuya, Kanonji-shi Kagawa 769-1602 (JP); TAKEUCHI, Mariko, Kanonji-shi Kagawa 769-1602 (JP)
(74) Representative: Peter, Julian
(86) International application number: PCT/JP2009/064491
(87) International publication number: WO 2010/032581

(56) References cited:
- EP-A1- 1 880 700
- EP-A2- 0 761 193
- WO-A2-2008/065953
- JP-A- 9 038 134
- JP-A- 9 099 006
- JP-A- 11 253 489
- JP-A- H11 253 489
- JP-A- 2001 252 303
- JP-A- 2005 279 077
- JP-A- 2007 082 890
- US-A- 5 916 206
- US-A1- 2006 025 746
- US-A1- 2006 036 227

## Description

### TECHNICAL FIELD

The present invention relates to disposable diapers and particularly to disposable diapers having elastic stretchability in front and rear waist regions to assure an appropriate fit to a wearer's body.

### RELATED ART

It is well known to provide front and rear waist regions with a plurality of strand-like waist elastic elements to improve a fit of the diaper to a wearer's body. For example, PATENT DOCUMENT 1 discloses a diaper characterized in that front and rear waist regions are respectively divided into three elastic zones provided with waist elastic elements having different levels of tensile stress, respectively. PATENT DOCUMENT 2 discloses a diaper characterized in that tensile stress in a front waist region is different from tensile stress in a rear waist region.
PATENT DOCUMENT 1: JP 3914673 B2
PATENT DOCUMENT 2: WO 2004/054482 A2

### DISCLOSURE OF THE INVENTION

### PROBLEM TO BE SOLVED BY THE INVENTION

According to the invention disclosed in PATENT DOCUMENT 1, each of the front and rear waist regions is divided into a first elastic zone circumferentially extending along a peripheral edge of a waist-opening, a second elastic zone which is adjacent to the first elastic zone and circumferentially extends and a third elastic zone which is adjacent to the second elastic zone and circumferentially extends wherein a correlative relationship of tensile stress in these three elastic zones are represented by the third elastic zone ≥ the second elastic zone > the first elastic zone. With this arrangement, the tensile stress in the first elastic zone is relatively low so that the waist-opening can be easily broadened to put the diaper on the wearer's body. In addition, both the front waist region and the rear waist region can express the tensile stress which is compatible with a body contour of the wearer, particularly, of an infant. In this way, a fit of the diaper as a whole can be improved.

According to the invention disclosed in PATENT DOCUMENT 2, the number of the waist elastic elements provided in the rear waist region is more than the number of the waist elastic elements provided in the front waist region and therefore the tensile stress of the elastic zone in the rear waist region is correspondingly higher than the tensile stress of the elastic zone in the front waist region. With the diaper put on the wearer's skin, in consequence, the fit of the rear waist region to the wearer's body is improved without anxiety that the front waist region might be excessively tightened under the effect of the waist elements. In this way, a feeling of tightness against the wearer can be reduced.

However, the wearer's body contour has different convexes and concaves depending on the lower ventral region, the ventral region and the buttocks. Even if the tensile stress is differentiated between the front waist region and the rear waist region or each of the front and rear waist regions is divided into three regions respectively having different levels of the tensile stress, it is impossible to set the levels of the tensile stress so that the diaper as a whole may be accurately compatible with the wearer's body contour.

If the levels of the tensile stress in the respective elastic zones are not compatible with the wearer's body contour, the diaper should be displaced during use and/or create an uncomfortable feeling of tightness against the wearer.

If the tensile stress is differentiated depending on the respective elastic zones merely by adjusting the number of the stretch ratio of the strand-like waist elastic elements, there is a possibility that the stress might be locally concentrated in the respective lines along which the strand-like elastic elements are laid and compression marks corresponding to the respective elastic elements might be left on the wearer's skin.

It is an object of the present invention to provide a disposable diaper improved to have tensile stress well compatible with the wearer's average body contour including convex and concave and thereby to eliminate an anxiety that the wearer might experience uncomfortable feeling of tightness.

### MEASURE TO SOLVE THE PROBLEM

According to the present invention, there is provided a disposable diaper having a longitudinal direction and a transverse direction, comprising a side facing the wearer's skin, a side facing away from the wearer's skin, a front waist region, a rear waist region, a crotch region extending between the front and rear waist regions, a waist-opening and a pair of leg-openings.

The present invention is characterized in that the front waist region is divided into a first elastic zone extending in the transverse direction along a peripheral edge of the waist-opening, a second elastic zone lying next to the first elastic zone and extending in the transverse direction and a third elastic zone lying next to the second elastic zone extending in the transverse direction between the second elastic zone and the crotch region; the rear waist region is divided into a fourth elastic zone extending in the transverse direction along the peripheral edges of the waist-opening, a fifth elastic zone lying next to the fourth elastic zone and extending in the transverse direction and a sixth elastic zone lying next to the fifth elastic zone and extending in the transverse direction between the fifth elastic zone and the crotch region; and correlation among values of tensile stress in the elastic zones can be represented by the sixth elastic zone > the second elastic zone > the third elastic zone ≥ the fifth elastic zone > the first elastic zone, and the sixth elastic zone > the second elastic zone > the third elastic zone ≥ the fifth elastic zone > the fourth elastic zone.

The present invention additionally includes embodiments as follows:
(1) The diaper comprises a front waist panel defining the front waist region, a rear waist panel defining the rear waist region and a liquid-absorbent chassis defining parts of the front and rear waist regions together with the crotch region and joined to the waist panels and containing a liquid-absorbent core.
(2) The front panel comprises a first inelastic sheet lying on the side facing away from the wearer's skin, a first elastic sheet laminated on the side of the first inelastic sheet facing the wearer's skin and a second elastic sheet laminated on a part of the first inelastic sheet and a part of the first elastic sheet; the rear waist panel comprises a second inelastic sheet lying on the side facing away from the wearer's skin, a third elastic sheet laminated on the side of the second inelastic sheet facing the wearer's skin and a fourth elastic sheet laminated on a part of the second inelastic sheet and the side facing the wearer's skin of a part of the third elastic sheet; the first elastic zone is defined by the first inelastic sheet and the second elastic sheet, the second elastic zone is defined by the first inelastic sheet, the first elastic sheet and the second elastic sheet, the third elastic zone is defined by the first inelastic sheet and the first elastic sheet, the fourth elastic zone is defined by the second inelastic sheet and the fourth elastic sheet, the fifth elastic zone is defined by the second inelastic sheet, the third elastic sheet and the fourth elastic sheet, the sixth elastic zone is defined by the second inelastic sheet, the third elastic sheet and the fourth elastic sheet; and a plurality of strand-like elastic elements extending in the transverse direction are sandwiched between the second inelastic sheet and the third elastic sheet in the sixth elastic zone.
(3) Values of tensile stress of the respective elastic zones at 65% of the maximum elongation are in a range of 20 to 40mN in the first elastic zone, in a range of 26 to 105mN in the second elastic zone, in a range of 25 to 65mN in the third elastic zone, in a range of 20 to 40mN in the fourth elastic zone, in a range of 25 to 65mN in the fifth elastic zone and in a range of 35 to 125mN in the sixth elastic zone.

### EFFECT OF THE INVENTION

According to the present invention, the front and rear waist regions are divided in six elastic zones having tensile stress differentiated one from another in accordance with average body contour of the wearer normally having gentle irregularities. With this unique arrangement, the diaper as a whole assures an appropriate fit and should not create an uncomfortable feeling of tightness against the wearer.

### BRIEF DESCRIPTION OF THE DRAWINGS

[FIG. 1] Fig. 1 is a perspective view of a disposable diaper.
[FIG. 2] Fig. 2 is a partially cutaway plan view showing the diaper of Fig. 1 in a flatly developed state.
[FIG. 3] Fig. 3 is an exploded perspective view of the diaper.
[FIG. 4] Fig. 4 is a sectional view taken along line IV-IV in Fig. 2.
[FIG. 5] Fig. 5 is a plan view of the diaper with a liquid-absorbent chassis removed.

### IDENTIFICATION OF REFERENCE NUMERALS USED IN THE DRAWINGS

- 10: disposable diaper
- 11: elastic waist panel
- 12: liquid-absorbent chassis
- 13: front waist region
- 14: rear waist region
- 15: crotch region
- 16: waist-opening
- 16a: peripheral edge of waist-opening
- 17a, 17b: leg-openings
- 18: front waist panel
- 19: rear waist panel
- 21: first inelastic sheet
- 22: first elastic sheet
- 23: second elastic sheet
- 24: second inelastic sheet
- 25: third elastic sheet
- 26: fourth elastic sheet
- 27: waist elastic elements
- 28: first elastic zone
- 29: second elastic zone
- 30: third elastic zone
- 31: fourth elastic zone
- 32: fifth elastic zone
- 33: sixth elastic zone
- 36: liquid-absorbent core
- X: transverse direction
- Y: longitudinal direction

### DETAILED DESCRIPTION OF THE PREFERRED EMBODIMENTS

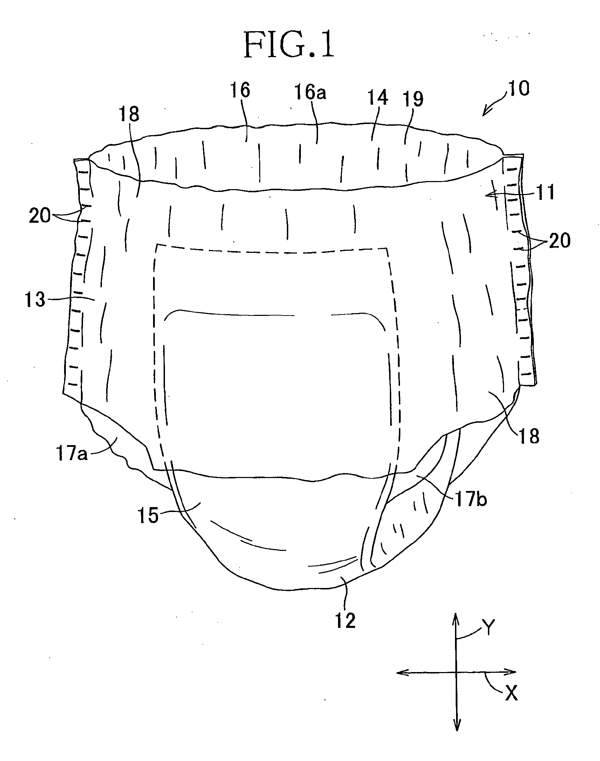

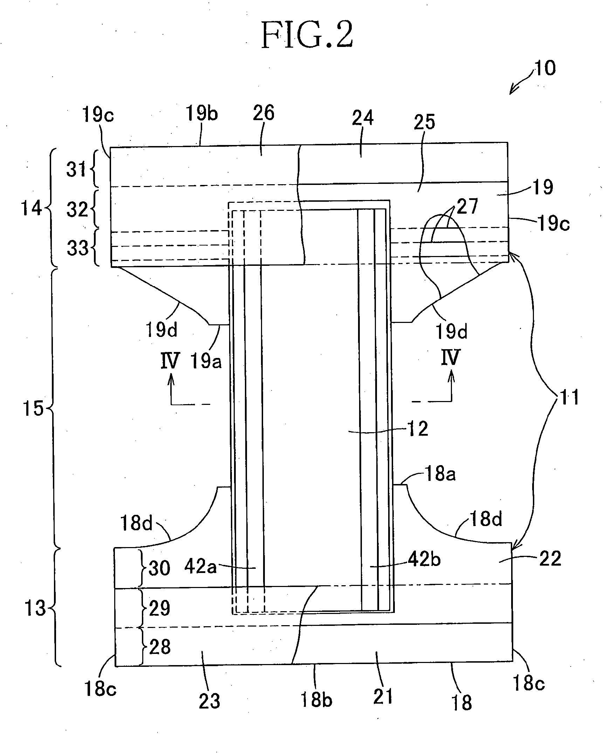

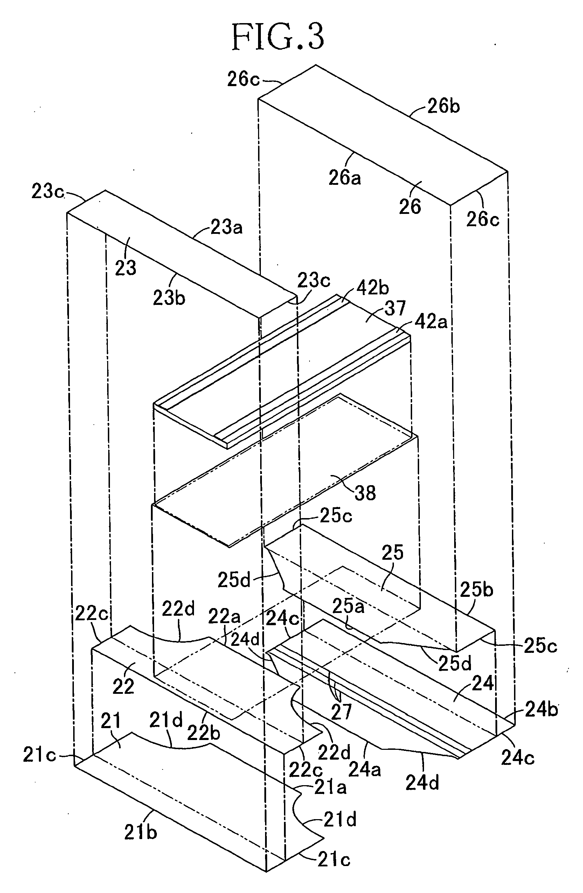

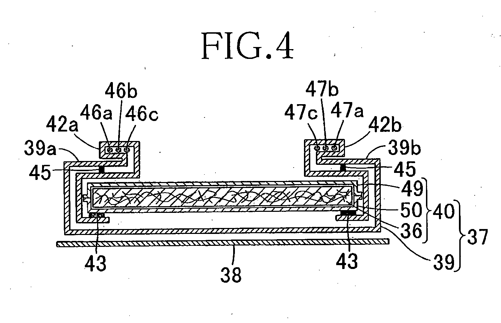

Fig. 1 is a perspective view of a diaper 10, Fig. 2 is a plan view showing a diaper 10 flatly developed in a longitudinal direction Y and a transverse direction X, Fig. 3 is an exploded perspective view of the diaper 10 and Fig. 4 is a sectional view taken along line V-V in Fig. 2. The term "elastic" used hereunder means that the zone in question is elastically stretchable. In contrast, the term "inelastic" means that the zone in question is elastically non-stretchable and/or inelastically stretchable.

Referring to Fig. 1, the diaper 10 has the longitudinal direction Y, the transverse direction X and comprises a side facing the wearer's skin and a side facing away from the wearer's skin, an annular elastic waist panel 11, a liquid-absorbent chassis 12 attached to the side facing the wearer's skin of the elastic waist panel 11, a front waist panel 13, a rear waist panel 14, a crotch region 15 extending between the front and rear waist regions 13, 14, a waist-opening 16 and a pair of leg-openings 17a, 17b. The waist-opening 16 is defined by the annular waist panel 11.

Referring to Figs. 1 and 2, the elastic waist panel 11 comprises a front waist panel 18 defining the front waist region 13 and a rear waist panel 19 defining the rear waist region 14. The front waist panel 18 has a generally trapezoidal shape contoured by an inner end 18a, an outer end 18b, respective outer side edges 18c, 18c and respective inner side edges 18d, 18d obliquely extending inward from the respective outer side edges 18c, 18c following downward concave curves, respectively. The rear waist panel 19 also has a generally trapezoidal shape contoured by an inner end 19a, an outer end 19b, respective outer side edges 19c, 19c and respective inner side edges 19d, 19d obliquely extending inward.

In a state of the diaper 10 formed into a pants-type, the respective outer side edges 18c, 18c of the front waist panel 18 are put flat and joined together with the respective outer side edges 19c, 19c of the rear waist panel 19 along side seams 20 arranged intermittently in the longitudinal direction Y by means of well known art, for example, various heat welding techniques such as heat embossing or ultrasonic sealing to define the waist-opening 16 and the front and rear waist panels 18, 19 cooperate with the liquid-absorbent chassis 12 to define a pair of leg-openings 17a, 17b (See Fig. 1).

Referring to Fig. 3, the front waist panel 18 comprises a generally trapezoidal first inelastic sheet 21 lying on the side facing away from the wearer's skin, a first elastic sheet 22 permanently bonded to the inner surface of the first inelastic sheet 21 and a second elastic sheet 23 laminated with the first elastic sheet 22 from above so as to sandwich the liquid-absorbent chassis 12.

The first inelastic sheet 21 has a generally trapezoidal shape contoured by an inner end 21a, an outer end 21b, opposite outer side edges 21c, 21c extending inward from the outer end 21b and opposite inner side edges 21d, 21d obliquely extending inward from the outer side edges 21c, 21c along downwardly concave lines, respectively. Such first inelastic sheet 21 defines the outer surface of the front waist panel 18.

The first elastic sheet 22 also has a generally trapezoidal shape similar to the first inelastic sheet 21 contoured by an inner end 22a, an outer end 22b, opposite outer side edges 22c, 22c and opposite inner side edges 22d, 22d wherein the outer side edges 22c, 22c are shorter than the outer side edges 21c, 21c of the first inelastic sheet 21. The first elastic sheet 22 is laminated on the first inelastic sheet 21 so that the inner end 22a, the opposite side edges 22c, 22c and the opposite inner side edges 22d, 22d thereof may respectively overlap the inner end 21a, part of the opposite outer side edges 21c, 21c and the opposite inner side edges 21d, 21d of the first inelastic sheet 21.

The second elastic sheet 23 has a rectangular shape which is relatively longer in the transverse direction X and contoured by an inner end 23a, an outer end 23b, opposite outer side edges 23c, 23c wherein the outer side edges 23c, 23c are shorter than those of the first inelastic sheet 21 wherein the outer end 23b overlaps the outer end 21b of the first inelastic sheet 21 and the opposite outer side edges 23c, 23c overlap a part of the opposite outer side edges 22c, 22c of the first elastic sheet 22 which overlap, in turn, part of the outer side edges 21c, 21c of the first inelastic sheet 21.

In a similar fashion, the rear waist panel 19 comprises a generally trapezoidal second inelastic sheet 24 lying on the side facing away from the wearer's skin, a third elastic sheet 25 permanently bonded to the inner surface of the second inelastic sheet 24 and a fourth elastic sheet 26 laminated on the third elastic sheet 25 from above so as to cover the liquid-absorbent chassis 12.

The second inelastic sheet 24 has a generally trapezoidal shape contoured by an inner end 24a, an outer end 24b, opposite outer side edges 24c, 24c extending inward from the outer end 24b and opposite inner side edges 24d, 24d obliquely extending between the outer side edges 24c, 24c and the inner end 24a following downward concave curves. The second inelastic sheet 24 defines the outer surface of the rear waist panel 19.

The third elastic sheet 25 has a generally trapezoidal shape similar to the second inelastic sheet 24 contoured by an inner end 25a, an outer end 25b, opposite outer side edges 25c, 25c and opposite inner side edges 25d, 25d wherein the opposite outer side edges 25c, 25c are shorter than the opposite outer side edges 24c, 24c of the second inelastic sheet 24. The inner end 25a, the opposite outer side edges 25c, 25c and the opposite inner side edges 25d, 25d are laminated to the inner end 24a, part of the opposite outer side edges 24c, 24c and the opposite inner side edges 24d, 24d, respectively.

The fourth elastic sheet 26 has a rectangular shape which is relatively longer in the transverse direction X and contoured by an inner end 26a, an outer end 26b, opposite outer side edges 26c, 26c wherein the outer side edges 26c, 26c respectively have same dimension as the second inelastic sheet 24 wherein the outer end 26b overlaps the outer end 24b of the second inelastic sheet 24, the opposite outer side edges 26c, 26c overlap the opposite outer side edges 24c, 24c of the second inelastic sheet 24, and the outer side edges 25c, 25c of the third elastic sheet 25 which, in turn, overlap the opposite side edges 24c, 24c of the second inelastic sheet 24.

In a lower section of the rear waist region 14 occupied by the end portion of the liquid-absorbent chassis 12, the second inelastic sheet 24 isprovidedon its inner surface with a plurality of strand-like waist elastic elements 27 extending in the transverse direction X between the opposite outer side edges 24c, 24c.

Referring again to Fig. 2, the front waist region 13 of this diaper 10 is divided into a first elastic zone 28 formed of the first inelastic sheet 21 and the second elastic sheet 23 and extending in the transverse direction X along the peripheral edge 16a of the waist-opening 16 (See Fig. 1), a second elastic zone 29 lying next to the first elastic zone 28 and comprising the first inelastic sheet 21, the first elastic sheet 22 and the second elastic sheet 23 laminated together, and a third elastic zone 30 lying next to the second elastic zone 29, extending in the transverse direction X between the second elastic zone 29 and the peripheral edges of the respective leg-openings and comprising the first inelastic sheet 21 and the first elastic sheet 22 laminated together.

The rear waist region 14 is divided into a fourth elastic zone 31 formed of the second inelastic sheet 24 and the fourth elastic sheet 26 and extending in the transverse direction X along the peripheral edges 16a of the waist-opening 16 (See Fig. 1), a fifth elastic zone 32 lying next to the fourth elastic zone 31 and comprising the second inelastic sheet 24, the third elastic sheet 25 and the fourth elastic sheet 26 laminated together, and a sixth elastic zone 33 lying next to the fifth elastic zone 32, extending in the transverse direction X between the fifth elastic zone 32 and the peripheral edges of the respective leg-openings and comprising the second inelastic sheet 24, the third elastic sheet 25 and the fourth elastic sheet 26 laminated together wherein the waist elastic elements 27 are sandwiched between the second inelastic sheet and the third elastic sheet 25.

While the first to third elastic zones 28, 29, 30 of the front waist region 13 have substantially the same dimension in the longitudinal direction Y in the illustrated embodiment, it is possible to differentiate this dimension for the respective elastic zones 28, 29, 30 in order to provide the respective elastic zones 28, 29, 30 with desired levels of tensile stress. This is true also for the fourth to sixth elastic zones 31, 32, 33 of the rear waist region 14.

Materials for the first and second inelastic sheets 21, 24 includes, but are not limited to, liquid-impervious plastic sheets or liquid-impervious fibrous nonwoven fabrics and materials for the first to fourth elastic sheets 22, 23, 25, 26 includes, but are not limited to, elastic fibrous nonwoven fabrics or inelastic nonwoven fabrics provided with a plurality of strand-like elastic elements to elasticize them or laminate sheets comprising two or more inelastic nonwoven fabric sheets sandwiching strand-like elastic elements or an elastic sheet.

For permanent bonding of such laminate sheets and the waist elastic elements 27, use of well known adhesives such as hot melt adhesives are suitable.

Now the correlation among values of tensile stress in the first to fourth elastic sheets 22, 23, 25, 26 will be described. The first elastic sheet 22 has the highest tensile stress which corresponds to or higher than the tensile stress supposed to be expressed if the third elastic sheet 25 and the fourth elastic sheet 26 are laminated together. The fourth elastic sheet 26 has tensile stress substantially equal to that of the second elastic sheet 23. The correlation among the tensile stress values of these elastic sheets may be represented by the first elastic sheet 22 ≥ the fourth elastic sheet 26 + the third elastic sheet 25 and the fourth elastic sheet 26 = the second elastic sheet 23.

Referring to Fig. 4, the liquid-absorbent chassis 12 includes a liquid-absorbent structure 37 containing therein the liquid-absorbent core 36 and an outer sheet 38 defining an outer surface of the crotch region 15 permanently bonded to a lower surface of the liquid-absorbent structure 37 with hot melt adhesives (not shown).

The liquid-absorbent structure 37 includes a liquid-impervious barrier sheet 39 and a liquid-absorbent pad 40 attached to the side of the barrier sheet 39 facing the wearer's skin as if the liquid-absorbent pad 40 is wrapped with the barrier sheet 39.

The barrier sheet 39 has opposite side edges 39a, 39b folded in a Z-shape and fixed to the lower surface of opposite side edges of the liquid-absorbent pad 40 with hot melt adhesives 43 so as to define an inverted Ω-shaped cross section. The opposite side edges 39a, 39b folded in a Z-shape define a pair of barrier cuffs 42a, 42b spaced from and opposed to each other in the transverse direction X and extending in the longitudinal direction Y.

The respective barrier cuffs 42a, 42b defined on both sides of the liquid-absorbent pad 40 have fixed edges formed by bonding the respective folded side edges 39a, 39a and respective inner surfaces thereof to themselves with hot melt adhesives 45 and sleeve-like free edges formed by distal folds of the respective side edges 39a, 39a. As viewed in the diaper 10 flatly developed, these free edges are folded outward of the opposite side edges 39a, 39a and lie generally above the respective fixed edges. These sleeve-like free edges are provided on the inner surfaces thereof with three elastic elements 46a, 46b, 46c; 47a, 47b, 47c, respectively, attached with hot melt adhesives (not shown). The elastic elements 46a, 46b, 46c; 47a, 47b, 47c are once stretched as the diaper 10 is put on the wearer's body and then contract again whereupon the free edges are spaced upward from the vicinity of the respective fixed edges. In consequence, leakage of body waste from the liquid-absorbent structure 37 can be reduced.

Referring to Figs. 2 and 3, the respective barrier cuffs 42a, 42b face the inner surface of the second elastic sheet 23 in the front waist region 13 and face the inner surface of the fourth elastic sheet 26 in the rear waist region 14. The respective barrier cuffs 42a, 42b are permanently bonded to the inner surfaces of these sheets 23, 26 with hot melt adhesives (not shown). With such arrangement, body waste discharged onto the liquid-absorbent structure 37 should not leak out of the front and rear waist regions 13, 14 even if such body waste flows toward the front and rear waist regions 13, 14.

While three elastic members 46a, 46b, 46c; 47a, 47b, 47c are attached within free edges of the respective barrier cuffs 47a, 47b according to the present embodiment, at least one elastic member may be attached within each of the free edges so far as, with the diaper 10 put on the wearer's body, the respective elastic members have a tensile stress necessary to put the free edges in close contact with the wearer's inguinal regions. The barrier cuffs 42a, 42b may be formed not of the barrier sheet 39 but of a separately prepared elastic sheet.

The liquid-absorbent pad 40 comprises a liquid-pervious inner sheet 49, a liquid-impervious outer sheet 50 and the liquid-absorbent core 36 sandwiched between these top- and outer sheets 49, 50.

The liquid-absorbent core 36 is formed of a mixture of fluff pulp, superabsorbent polymer (SAP) particles and, if necessary, heat-sealable staple fiber and preferably wrapped with a liquid-dispersant sheet such as tissue paper for shape retention. To improve the shape retention and the dispersibility, the liquid-absorbent core 28 is compressed generally in an hourglass-shape and, in consequence, has stiffness higher than the other sheet members. In other words, the liquid-absorbent core 28 is high rigid or semirigid in comparison with the other sheet members.

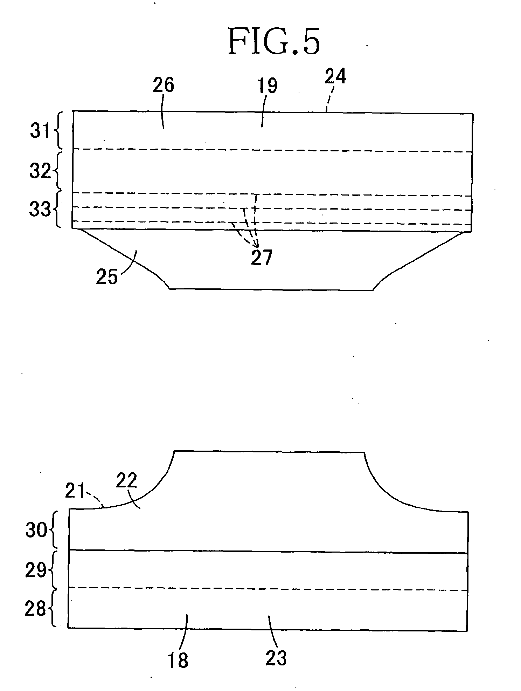

Fig. 5 is a plan view showing the elastic waist panel 11 alone with the liquid-absorbent chassis 12 removed for convenience of explanation.

As has previously been described, the front and rear waist regions 13, 14 are divided into the first to sixth elastic zones 28, 29, 30, 31, 32, 33 wherein the values of tensile stress expressed in the respective elastic zones depend on combination of the above-mentioned correlation among values of tensile stress of the first to fourth elastic sheets 22, 23, 25, 26, the manner in which the respective sheets are laminated one on another and the arrangement of the waist elements.

Specifically, the second inelastic sheet 24, the third elastic sheet 25 and the fourth elastic sheet 26 are laminated and the waist elastic elements 27 are sandwiched between the second inelastic sheet 24 and the third elastic sheet 25 in the sixth elastic zone 33. In consequence, the sixth elastic zone 33 has tensile stress higher than the second elastic zone 29 formed by the first elastic sheet 22 and the second elastic sheet 23 laminated together.

In the third elastic zone 30, the first inelastic sheet 21 and the first elastic sheet 22 are laminated together and the tensile stress in this zone 30 is lower than that in the second elastic zone 29. In the fifth elastic zone 32, the second inelastic sheet 24, the third and fourth elastic sheets 25, 26 are laminated together and the correlation of values of tensile stress in the respective elastic sheets is represented by the first elastic sheet 22 ≥ the fourth elastic sheet 26 + the third elastic sheet 25. Therefore, the fifth elastic zone 32 has tensile stress equal to or lower than that of the third elastic zone 30.

The first elastic zone 28 is formed of the first inelastic sheet 21 and the second elastic sheet 23, the fourth elastic zone 31 is formed of the second inelastic sheet 24 and the fourth elastic sheet 26 and the second elastic sheet 23 has tensile stress substantially equal to tensile stress in the fourth elastic sheet 26. Therefore, both the first elastic zone 28 and the fourth elastic zone 31 have tensile stress lower than tensile stress in the fifth elastic zone 32.

In conclusion, the correlation among values of tensile stress in the respective elastic zones can be represented by the sixth elastic zone 33 > the second elastic zone 29 > the third elastic zone 30 ≥ the fifth elastic zone 32 > the first elastic zone 28, and the sixth elastic zone 33 > the second elastic zone 29 > the third elastic zone 30 ≥ the fifth elastic zone 32 > the fourth elastic zone 31.

From the above-described correlation, the correlation between the front waist region 13 as a whole and the rear waist region 14 as a whole can be derived as follows: the rear waist region 14 > the front waist region 13, i.e., the fourth elastic zone 31 + the fifth elastic zone 32 + the sixth elastic zone 33 > the first elastic zone 28 + the second elastic zone 29 + the third elastic zone 30.

Assumed that the front and rear waist regions 13, 14 are divided into an upper zone (the first elastic zone 28 and the fourth elastic zone 31), a middle zone (the second elastic zone 29 and the fifth elastic zone 32) and a lower zone (the third elastic zone 30 and the sixth elastic zone 33), a correlation among values of tensile stress in these three zones can be represented by the lower zone > the middle zone > the upper zone, i.e., the third elastic zone 30 + the sixth elastic zone 33 > the second elastic zone 29 + the fifth elastic zone 32 > the first elastic zone 28 + the fourth elastic zone 31.

The average wearer's body contour includes various degrees of convex and concave such as concave in the lower ventral region, convex in the ventral region and significant convex in the buttock region. Correspondingly, various degrees of tensile stress are required, depending on the various zones in the front and rear waist regions 13, 14, to assure appropriate fit of the diaper 10 to the wearer's body depending on the various zones in the front and rear waist regions 13, 14. In other words, if the front and rear waist regions 13, 14 are designed to have an uniform tensile stress, it will be difficult to assure a desired fit of the diaper 10 to the wearer's body. In addition, the diaper 10 should be readily displaced from its proper position and should create an uncomfortable feeling of tightness against the wearer.

To avoid such problems, according to the present invention, the front and rear waist regions 13, 14 are divided into six zones having differentiated values of tensile stress.

Specifically, in the front waist region 13, the second elastic zone 29 is formed so as to face a region of the wearer's body convexly extending outward over the ventral region, the third elastic zone 30 is formed so as to face the wearer's thighs and the first elastic zone 28 is formed so as to face the ventral region significantly protruding outward.

In the rear waist region 14, the sixth elastic zone 33 is formed so as to face the wearer's buttocks, the fifth elastic zone 32 is formed so as to face the region concavely extending from the buttocks toward the lumbar area and the fourth elastic zone 31 is formed so as to face the lumbar area.

Considering the average wearer's body contour, the lower zone (the third elastic zone 30 and the sixth elastic zone 33) of the front and rear waist regions 13, 14 facing the wearer's sidebone may be put in close contact with the wearer's body to assure the lower zone to be caught by the wearer's sidebone and thereby to position-keep the diaper without causing a displacement as serious as disturbing normal use of the diaper.

However, the higher the tensile stress in the lower zone, the higher the possibility that the wearer's thighs should be excessively compressed, creating an uncomfortable feeling of tightness against the wearer. Excessively high tensile stress in the lower zone will necessarily make it difficult to pull the lower zone in the transverse direction and cause another problem that an operability of putting the diaper on the wearer' s body should be deteriorated and a compression mark might be left on the wearer's skin.

According to the present invention, the tensile stress in the sixth elastic zone 33 facing the wearer's sidebone and being resistant to displacement is set to the highest value, the tensile stress in the third elastic zone 30 horizontally opposed to the sixth elastic zone 33 in the front-back direction is set to be lower than the tensile stress in the second elastic zone 29 overlying the third elastic zone 30 and the tensile stress in the fifth elastic zone 32 horizontally opposed to the second elastic zone 29 in the front-back direction and overlying the sixth elastic zone 33 is set to be equal to or lower than the tensile stress in the third elastic zone 30. Consequently, the correlation among values of tensile stress in the middle zones and the lower zones of the front and rear waist regions 13, 14 can be represented by the sixth elastic zone 33 > the second elastic zone 29 > the third elastic zone 30 ≥ the fifth elastic zone 33. The sixth elastic zone 33 having the highest tensile stress in the rear waist region 14 and the second elastic zone 29 having the highest tensile stress in the front waist region 13 are located asymmetrically in the vertical direction and, in consequence, the wearer' s body should not be locally compressed. In this way, the diaper 10 as a whole assures an appropriate fit with generation of compression marks and adverse affection on diaper handling being restricted as effectively as possible.

More specifically, on the assumption that the elastic zones horizontally facing each other in the front-back direction of the diaper 10, for example, the second elastic zone 29 and the fifth elastic zone 32 horizontally facing each other have the highest tensile stress in the front and rear waist regions 13, 14, respectively, if the diaper 10 is displaced downward from the level at which the diaper 10 will be stably retained on the wearer's body, the middle zone of the front waist region 13 will be gathered downward into the zone facing the wearer's thighs and eventually the front and rear waist regions 13, 14 as a whole should slip down. In consideration of such problem, according to the present invention, the second elastic zone 29 and the sixth elastic zone 33 having the highest tensile stress in the front and rear waist regions 13, 14, respectively, are unevenly opposed to each other, i.e., displaced from each other in the vertical direction. With such an arrangement, even if the diaper 10 is displaced from the desired position at which the diaper 10 is stably retained and the middle zone of the front waist region 13 is gathered downward, the rear waist region 14 is kept in close contact with the wearer's body. In this way, the diaper 10 should be significantly slipping down from the desired position.

According to the present invention, the elastic sheet members are used together with the strand-like elastic elements to set the tensile stress depending on the respective elastic zones. With Such combined usage should not create a feeling of uncomfortable tightness and compression marks against the wearer's skin. This is because the combined usage of the elastic sheet members and the strand-like elastic elements appropriately distribute the stress in comparison with the case in which the strand-like elastic elements are exclusively used and thereby the stress is locally adjusted to be high or low.

While the elastic sheets respectively having different values of tensile stress are laminated to adjust the tensile stress in the respective elastic zones according to the present embodiment, it is possible to fold back one and same elastic sheet on itself so that the folded elastic sheet may have tensile stress higher than that of the same elastic sheet before it has been folded back on itself. It is also possible to replace the strand-like waist elastic elements 27 by elastic sheets. It is also possible to subject the elastic nonwoven fabric used as the elastic sheet to appropriate treatment such as heat press or heat seal cut in order to reduce elasticity thereof. Furthermore, it is also possible to stick an inelastic member such as inelastic nonwoven fabrics or inelastic plastic films to the elastic sheet or to coating the elastic sheet with hot melt adhesives and thereby to lower the elasticity thereof.

Values of tensile stress of the respective elastic zones at 65% of the maximum elongation are preferably in a range of 20 to 40mN in the first elastic zone, in a range of 26 to 105mN in the second elastic zone, in a range of 25 to 65mN in the third elastic zone, in a range of 20 to 40mN in the fourth elastic zone, in a range of 25 to 65mN in the fifth elastic zone and in a range of 35 to 125mN in the sixth elastic zone.

Each tensile stress of the respective elastic zones in the front and rear waist regions is measured by a method as follows.

The front and rear waist regions are peeled off each other along the seam arrays of the diaper 10 to develop the diaper as shown in Fig. 2 and the respective elastic zones are stretched to measure a length of each elastic zone as the length at the maximum elongation in the transverse direction X. Then, the front waist region 13 as a whole and the rear waist region 14 as a whole are cut off from the diaper 10 and the respective elastic zones are cut off from the front and rear waist regions having been cut off to obtain test pieces of the respective elastic zones. Region width (i.e., dimension in the longitudinal direction Y of the diaper 10) is measured for each of the test pieces. The test piece in a contracted state is set between chucks (chuck-distance is 100mm and appropriately adjusted depending on the test piece) of Tensile Tester (manufactured by Shimadzu Corporation) and the test piece is stretched in the same direction as the transverse direction X of the diaper 10 at a tension rate of 100mm/min. A load (mN) at a length corresponding to 65% of the length at the maximum elongation is measured and the tensile stress is calculated by measured value (mN) ÷ region width (mm) for the respective elastic zones.

As the component members of the diaper 10 such as the elastic waist panel 11 and the liquid-absorbent chassis 12, various types of material widely used in the related technical field may be selectively used so far as particular materials are not specified as such component members in this specification. While the front and rear waist regions 13, 14 are formed of the front and rear waist panels 18, 19 according to the present embodiment, it is possible to form the front and rear waist regions 13, 14 by a continuous sheet member integrally forming the outer shape of the diaper as a whole. While the elastic waist panel 11 and the liquid-absorbent chassis 12 are separately prepared according to the illustrated embodiment, it is also possible to sandwich the liquid-absorbent core between a pair of hourglass-shaped elastic waist panels. The present invention is applicable not only to pants-type disposable diapers but also to open-type disposable diapers in which the side edges of the front and rear waist regions are not previously joined together.

## Claims

1. A disposable diaper having a longitudinal direction and a transverse direction comprising a side facing the wearer's skin, a side facing away from the wearer's skin, a front waist region, a rear waist region, a crotch region extending between said front and rear waist regions, a waist-opening and a pair of leg-openings, said disposable diaper being **characterized in that**:
said front waist region is divided into a first elastic zone extending in said transverse direction along a peripheral edge of said waist-opening, a second elastic zone lying next to said first elastic zone and extending in said transverse direction and a third elastic zone lying next to said second elastic zone extending in said transverse direction between said second elastic zone and said crotch region;
said rear waist region is divided into a fourth elastic zone extending in said transverse direction along the peripheral edges of said waist-opening, a fifth elastic zone lying next to said fourth elastic zone and extending in said transverse direction and a sixth elastic zone lying next to said fifth elastic zone and extending in said transverse direction between said fifth elastic zone and said crotch region; and
correlation among values of tensile stress in said elastic zones can be represented by the sixth elastic zone > the second elastic zone > the third elastic zone ≥ the fifth elastic zone > the first elastic zone and the sixth elastic zone > the second elastic zone > the third elastic zone ≥ the fifth elastic zone > the fourth elastic zone.

2. The diaper defined by Claim 1, wherein said diaper comprises a front waist panel defining said front waist region, a rear waist panel defining said rear waist region and a liquid-absorbent chassis defining parts of said front and rear waist regions together with said crotch region and joined to said waist panels and containing a liquid-absorbent core.

3. The diaper defined by Claim 2, wherein said front panel comprises a first inelastic sheet lying on said side facing away from the wearer's skin, a first elastic sheet laminated on the side of said first inelastic sheet facing the wearer's skin and a second elastic sheet laminated on a part of said first inelastic sheet and a part of said first elastic sheet;
said rear waist panel comprises a second inelastic sheet lying on said side facing away from the wearer's skin, a third elastic sheet laminated on said side of said second inelastic sheet facing the wearer' s skin and a fourth elastic sheet laminated on a part of said second inelastic sheet and said side facing the wearer's skin of a part of said third elastic sheet;
said first elastic zone is defined by said first inelastic sheet and said second elastic sheet, said second elastic zone is defined by said first inelastic sheet, said first elastic sheet and said second elastic sheet, said third elastic zone is defined by said first inelastic sheet and said first elastic sheet, said fourth elastic zone is defined by said second inelastic sheet and said fourth elastic sheet, said fifth elastic zone is defined by said second inelastic sheet, said third elastic sheet and said fourth elastic sheet, said sixth elastic zone is defined by said second inelastic sheet, said third elastic sheet and said fourth elastic sheet; and
a plurality of strand-like elastic elements extending in said transverse direction are sandwiched between said second inelastic sheet and said third elastic sheet in said sixth elastic zone.

4. The diaper defined by any one of Claims 1 through 3, wherein values of tensile stress of the respective elastic zones at 65% of the maximum elongation are in a range of 20 to 40mN in the first elastic zone, in a range of 26 to 105mN in the second elastic zone, in a range of 25 to 65mN in the third elastic zone, in a range of 20 to 40mN in the fourth elastic zone, in a range of 25 to 65mN in the fifth elastic zone and in a range of 35 to 125mN in the sixth elastic zone.

## Patentansprüche

1. Wegwerfwindel mit einer Längsrichtung und einer Querrichtung umfassend eine der Haut des Trägers zuweisende Seite, eine der Haut des Trägers abweisende Seite, einen vorderen Taillenbereich, einen hinteren Taillenbereich, einen sich zwischen dem vorderen Taillenbereich und dem hinteren Taillenbereich erstreckenden Schrittbereich, eine Taillenöffnung und ein Paar Beinöffnungen, wobei die Wegwerwinedl **dadurch gekennzeichnet ist, dass**:
der vordere Taillenbereich in einen ersten elastischen Abschnitt, der sich in Querrichtung entlang einer Außenkante der Taillenöffnung erstreckt, einen zweiten elastischen Abschnitt, der angrenzend an den ersten elastischen Abschnitt liegt und sich in Querrichtung erstreckt, und einen dritten elastischen Abschnitt, der in Querrichtung angrenzend an den zweiten elastischen Abschnitt zwischen dem zweiten elastischen Abschnitt und dem Schrittbereich liegt, unterteilt ist;
der hintere Taillenbereich in einen vierten elastischen Abschnitt, der sich in Querrichtung entlang Außenkanten der Taillenöffnung erstreckt, einen fünften elastischen Abschnitt, der angrenzend an den vierten elastischen Abschnitt liegt und sich in Querrichtung erstreckt, und einen sechsten elastischen Abschnitt, der in Querrichtung angrenzend an den fünften elastischen Abschnitt zwischen dem fünften elastischen Abschnitt und dem Schnittbereich liegt, unterteilt ist; und
Korrelationen zwischen Werten der Zugspannung der elastischen Abschnitte können repräsentiert werden durch der sechste elastische Abschnitt > der zweite elastischen Abschnitt > der dritte elastische Abschnitt ≥ der fünfte elastische Abschnitt > der erste elastische Abschnitt und der sechste elastische Abschnitt > der zweite elastische Abschnitt > der dritte elastische Abschnitt ≥ der fünfte elastische Abschnitt > der vierte elastische Abschnitt.

2. Windel nach Anspruch 1, wobei die Windel ein den vorderen Taillenbereich bestimmendes vorderes Taillenpanel, ein den hinteren Taillenbereich bestimmendes hinteres Taillenpanel und ein flüssigkeitsabsobierendes Chassis aufweist, das zusammen mit dem Schrittbereich Teile der vorderen und hinteren Taillenbereiche bestimmt und an den Taillenpanelen befestigt ist und einen flüssigkeitsaborbierenden Kern aufweist.

3. Windel nach Anspruch 2, wobei das vordere Panel eine erste nicht-elastische Lage, die auf der der Haut des Trägers abweisenden Seite liegt, eine erste elastische Lage, die auf der ersten nicht-elastischen Lage auf der der Haut des Trägers zuweisenden Seite laminiert ist, und eine zweite elastische Lage, die auf einen Teil der ersten nicht-elastischen Lage und einen Teil der ersten elastischen Lage laminiert ist, umfasst;
das hintere Panel eine zweite nicht-elastische Lage, die auf der der Haut des Trägers abweisenden Seite liegt, eine dritte elastische Lage, die auf der zweiten nicht-elastischen Lage auf der der Haut des Trägers zuweisenden Seite laminiert ist, und eine vierte elastische Lage, die auf einen Teil der zweiten nicht-elastischen Lage und die der Haut des Trägers zuweisende Seite eines Teils der dritten elastischen Lage laminiert ist, umfasst;
der erste elastische Abschnitt durch die erste nicht-elastische Lage und die zweite elastische Lage bestimmt ist, der zweite elastische Abschnitt durch die erste nicht-elastische Lage, die erste elastische Lage und die zweite elastische Lage bestimmt ist, der dritte elastische Abschnitt durch die erste nicht-elastische Lage und die erste elastische Lage bestimmt ist, der vierte elastische Abschnitt durch die zweite nicht-elastische Lage und die vierte elastische Lage bestimmt ist, der fünfte elastische Abschnitt durch die zweite nicht-elastische Lage, die dritte elastische Lage und die vierte elastische Lage bestimmt ist, der sechste elastische Abschnitt durch die zweite nicht-elastische Lage, die dritte elastische Lage und die vierte elastische Lage bestimmt ist; und
sich eine Vielzahl fadenartiger elastischer Elemente, die sich in Querrichtung erstrecken, zwischen der zweiten nicht-elastischen Lage und der dritte elastischen Lage in dem sechsten elastischen Abschnitt sandwichartig aufgenommen ist.

4. Windel nach einem belibeigen der Ansprüche 1 bis 3, wobei Werte der Zugspannungen der jeweiligen elastischen Abschnitte bei 65% der maximalen Verlängerung in einem Bereich von 20 bis 40 mN in dem ersten elastischen Abschnitt, in einem Bereich von 26 bis 105 mN in dem zweiten elastischen Abschnitt, in einem Bereich von 25 bis 65 mN in dem dritten elastischen Abschnitt, in einem Bereich von 20 bis 40 mN in dem vierten elastischen Abschnitt, in einem Bereich von 25 bis 65 mN in dem fünften elastischen Abschnitt und in einem Bereich von 35 bis 125 mN in dem sechsten elastischen Abschnitt liegen.

## Revendications

1. Couche-culotte jetable ayant une direction longitudinale et une direction transversale, comprenant une côté faisant face à la peau du porteur et une côté opposée de la peau du porteur, une région de ceinture avant, une région de ceinture arrière, et une région d'entrejambe s'étendant entre la région de ceinture avant et la région de ceinture arrière, une ouverture de ceinture et une paire d'ouvertures pour les jambes, dans lequel la couche-culotte jetable est **caractérisé en ce que**:
la région de ceinture avant est divisée dans une première partie élastique qui se prolonge dans la direction transversale le long d'un bord extérieur de l'ouverture de taille, une deuxième partie élastique, qui est adjacente à la première section élastique et se prolongeant dans la direction transversale, et une troisième partie élastique adjacente dans la direction transversale à la deuxième partie élastique entre la seconde partie élastique et la région d'entrejambe;
la région de ceinture arrière est divisée d'une quatrième section élastique s'étendant transversalement le long de bords extérieurs de l'ouverture de taille, une cinquième partie, qui est adjacente à la quatrième partie élastique et se prolongeant dans la direction transversale et une sixième partie élastique orientée dans le sens transversal adjacent à la cinquième partie élastique est arrangée entre la partie élastique, et la région d'entrejambe; et
les corrélations entre les valeurs de la contrainte de traction des parties élastiques peuvent être représentés par la sixième partie élastique > la deuxième partie élastique > la troisième partie élastique ≥ la cinquième section élastique > la première partie élastique et la sixième partie élastique > la deuxième partie élastique > la troisième élastique section ≥ cinquième partie élastique > la quatrième partie élastique.

2. Couche-culotte selon la revendication 1, dans lequel la couche-culotte ayant un panneau de ceinture avant définissant la région de ceinture avant, un panneau de ceinture arrière définissant une région de ceinture arrière et un châssis absorbant les liquides qui détermine avec la région d'entre-jambes des parties des régions de ceinture avant et arrière et qui est fixés aux panneaux de la ceinture et ayant un noyau absorbant aux liquides.

3. Couche-culotte selon la revendication 2, dans lequel le panneau avant comprenant une première couche non élastique, qui se trouve opposée du côté de la peau de l'utilisateur, une première couche élastique qui est stratifiée sur la première couche non-élastique sur le côté faisant face à la peau de l'utilisateur, et une seconde couche élastique stratifié sur une partie de la première couche non-élastique et sur une partie de la première couche élastique;
le panneau arrière comprends une deuxième couche non-élastique, qui se trouve opposée du côté de la peau de l'utilisateur, une troisième couche élastique laminée sur la deuxième couche non-élastique sur le côté faisant face à la peau du porteur, et une quatrième couche élastique, qui est stratifiée sur une partie de la seconde couche non-élastique et sur une partie de la troisième couche élastique qui faisant face à la côté du peau de l'utilisateur ;
la première partie élastique est déterminée par la première couche non-élastique et la deuxième couche élastique,
la seconde partie élastique est déterminée par la première couche non-élastique, la première couche élastique et la deuxième couche élastique,
la troisième partie élastique est déterminée par la première couche non-élastique et la première couche élastique,
la quatrième partie élastique est déterminée par la deuxième couche non-élastique et la quatrième couche élastique,
la cinquième partie élastique est déterminée par la deuxième couche non-élastique, la troisième couche élastique et le quatrième couche élastique,
la sixième partie élastique est déterminée par la deuxième couche non-élastique, la troisième couche élastique et la quatrième couche élastique; et une pluralité d'éléments semblables à des fils élastiques qui se prolongent dans la direction transversale, sont arrangées en manière de sandwich entre la deuxième couche non-élastique et la troisième couche élastique dans la sixième partie élastique.

4. Couche-coulotte selon l'une quelconque des revendications 1 à 3, dans lequel les valeurs de la contrainte de traction des parties élastiques respectives à 65% de l'extension maximale sont dans la domaine de 20 à 40 mN à la première section élastique, dans une domaine de 26 à 105 mN à la deuxième partie élastique, dans une domaine de 25 à 65 mN à la troisième partie élastique, dans une domaine de 20 à 40 mN à la quatrième partie élastique, dans une domaine de 25 à 65 mN à la cinquième partie élastique et dans une domaine de 35-125 mN dans la sixième partie élastique.
